# EUROPÄISCHE PATENTSCHRIFT

(11) **EP 1 102 738 B1**
(45) Veröffentlichungstag und Bekanntmachung des Hinweises auf die Patenterteilung: **04.12.2002**
(21) Anmeldenummer: 99936531.5
(22) Anmeldetag: 13.07.1999
(51) Int. Cl.: C07C 51/08, C07C 63/331

(54) **VERFAHREN ZUR SAUREN ODER ALKALISCHEN VERSEIFUNG VON BIPHENYLNITRILEN**
METHOD FOR THE ACIDIC OR ALKALINE SAPONIFICATION OF BIPHENYL NITRILES
PROCEDE DE SAPONIFICATION ACIDE OU ALCALINE DE BIPHENYLNITRILES

(30) Priorität: 15.07.1998 DE 19831817
(43) Veröffentlichungstag der Anmeldung: 30.05.2001
(73) Patentinhaber: Lehmann, Bernd, Dr., 78465 Konstanz (DE)
(72) Erfinder: LEHMANN, Bernd, D-78465 Konstanz (DE); BREDLO, Lars, D-78354 Sipplingen (DE)
(86) Internationale Anmeldenummer: EP9904955
(87) Internationale Veröffentlichungsnummer: WO00003964

(56) Entgegenhaltungen:
- K.A. CIRIGOTTIS ET AL.: "Studies on the Hurtley Reaction" AUSTRALIAN JOURNAL OF CHEMISTRY, Bd. 27, Nr. 10, 1974, Seiten 2209-2228, XP002121411
- A. SIEGEL ET AL.: "Über die Darstellung von m-Hydroxybenzonitrilen durch die Reaktion von Alkalicyaniden mit Chinolacetaten" MONATSH. CHEM., Bd. 88, 1957, Seiten 228-233, XP002121412
- H.O. HOUSE ET AL.: "The rotational barrier in 1,8-diarylnaphtalenes" JOURNAL OF ORGANIC CHEMISTRY, Bd. 35, Nr. 6, 1970, Seiten 1815-1819, XP002121413

## Beschreibung

Die vorliegende Erfindung betrifft ein Verfahren zur sauren oder alkalischen Verseifung von Biphenylnitrilen, wobei die Verseifung in Wasser und unter Druck ausgeführt wird.

Es sind bereits verschiedene Verfahren zur Synthese von Biphenylcarbonsäuren bekannt.

Die Dokumente K. A. Cirigottis et al.: "Studies on the Hurtley Reaction" Australian Journal of Chemistry, Bd. 27, Nr. 10, 1974, Seiten 2209-2228, A. Siegel et al.: "Über die Darstellung von m-Hydroxybenzonitrilen durch die Reaktion von Alkalicyaniden mit Chinolacetaten" Monatsh. Chem., Bd. 88, 1957, Seiten 228 bis 233, H. O. House et al.: "The rotational barrier in 1,8-diarylnaphtalenes" Journal of Organic Chemistry, Bd. 35, Nr. 6, 1970, Seiten 1815 bis 1819, offenbaren Verfahren zur Verseifung von Biphenylnitrilen in Wasser.

In DE-C-19632643 ist die Umsetzung von Aryl-Grignard-Verbindungen mit Bromarylcarbonsäuren in Tetrahydrofuran in Gegenwart von Triphenylphosphin beschrieben, die zu Biphenylcarbonsäuren führt.

In Synthesis (1995) 41-43 wird die Umsetzung von 2,6-Di-tert-butyl-4-methylphenyl-2-methoxybenzoat mit 4-Methylphenylmagnesiumbromid in Diethylether/Benzol zum entsprechenden 4'-Methylbiphenyl-2-carboxylat beschrieben, das durch Umesterung mittels Natriummethoxid in Toluol/1-Methyl-2-pyrrolidon und anschließende Hydrolyse in Wasser/Ethanol zur 4'-Methylbiphenyl-2-carbonsäure umgesetzt wird.

Da Carbonsäurenitrile durch eine Vielzahl synthetischer Methoden leicht zugänglich sind (siehe beispielsweise WO 97/30970; CHIMICA OGGI/Chemistry Today, März/April 1998, S. 18 - 23; Negishi Coupling, E.-I. Negishi, A. O. King, J. Org. Chem. 42 (1977) 1821; Meyers Coupling, A. I. Meyers, E. D. Mihelich, J. Am. Chem. Soc. 97 (1975) 7383; Suzuki Coupling, US-A-5130439 und DE-C-19607135), stellt die Verseifung von Nitrilen zu Carbonsäuren eine alternative, präparative Methode dar.

Jedoch sind aromatische Nitrile unter anderem wegen ihrer Unlöslichkeit in Wasser relativ beständig gegenüber Hydrolyse. Um eine homogenere Mischung zu erhalten wird daher bei der alkalischen Verseifung oft in alkoholischer Lösung gearbeitet und bei der sauren Hydrolyse oft Eisessig als Lösungsvermittler eingesetzt.

In JP-A-100 77 244 (Chemical Abstracts 128 (1998) 230140) wird die Hydrolyse von Arylbenzonitrilen zu Arylbenzoesäuren in Gegenwart einer Alkalimetall- oder Erdalkalimetallbase und organischen Lösungsmitteln beschrieben. Dieses Verfahren ergibt Biphenyl-2-carbonsäure in 79 % Ausbeute, wenn Biphenyl-2-nitrit 25 Stunden mit Natriumhydroxid in wäßrigem Methanol unter Rückfluß erwärmt wird.

In diesem Verfahren werden organische Lösungsmittel verwendet, die nach der Umsetzung entsorgt werden müssen. Außerdem führt dieses Verfahren nur in relativ niedriger Ausbeute zum gewünschten Zielprodukt.

Aufgabe der vorliegenden Erfindung ist es daher, die vorstehend beschriebenen Nachteile des Stands der Technik zu vermeiden, das heißt, ein einfach durchführbares Verfahren bereitzustellen, das ausgehend von leicht verfügbaren Ausgangsstoffen ohne Verwendung von organischen Lösungsmitteln mit hoher Ausbeute und Selektivität zum Zielprodukt führt.

Überraschenderweise wurde nun gefunden, daß diese Ziele erreicht werden können, indem Biphenylnitrile in Wasser unter Druck alkalisch oder sauer verseift werden.

Das heißt, die vorliegenden Erfindung stellt ein Verfahren zur alkalischen oder sauren Verseifung von Biphenylnitrilen bereit, dadurch gekennzeichnet, daß die Verseifung in Wässer (als Lösungsmittel) und unter einem Druck von 3 bis 20 bar ausgeführt wird.

Dieses Verfahren ergibt beispielsweise bei der Umsetzung von 4'-Methylbiphenyl-2-nitril mit Natriumhydroxid als Base nach üblicher Aufarbeitung 4'-Methylbiphenyl-2-carbonsäure in einer Ausbeute von 98,7 % mit einer Reinheit von 99,5 %.

Es werden keine organischen Lösungsmittel eingesetzt, die nach der Umsetzung entsorgt werden müssen. Die Ausgangsstoffe sind billig und leicht verfügbar. Der bei diesem Verfahren entstehende Ammoniak ist frei von Lösungsmitteln und kann daher verwertet werden.

Bevorzugte Ausführungsformen der vorliegenden Erfindung sind in den Ansprüchen dargestellt. Insbesondere hat es sich bewährt, die Verseifung von 4'-Methylbiphenyl-2-nitril mit Natriumhydroxid als Base bei einer Temperatur von 150 bis 155 °C und einem Druck von 3 bis 5 bar auszuführen.

Wegen weiterer Vorteile des erfindungsgemäßen Verfahrens kann auf die folgende Beschreibung und das Ausführungsbeispiel verwiesen werden.

Gemäß der vorliegenden Erfindung wird sowohl bei der Umsetzung als auch bei der Aufarbeitung des Produkts nur Wasser als Lösungsmittel eingesetzt, so daß die Aufarbeitung oder kostspielige Entsorgung organischer Lösungsmittel entfällt.

Das erfindungsgemäße Verfahren ist allgemein für Biphenylnitrile, die beliebig substituiert sein können, geeignet, deren Substituenten unter alkalischen und/oder sauren Bedingungen stabil sind bzw. Schutzgruppen darstellen, die unter den Reaktionsbedingungen abgespalten werden sollen (beispielsweise kann im Falle der Verseifung mit Bromwasserstoffsäure eine Methoxygruppe zu einer Hydroxygruppe umgesetzt werden). Die Herstellung von für das erfingungsgemäße Verfahren geeigneten Biphenylnitrilen ist beispielsweise in WO 97/30970; CHIMICA OGGI/Chemistry Today, März/April 1998, S. 18 - 23; Negishi Coupling, E.-I. Negishi, A. O. King, J. Org. Chem. 42 (1977) 1821; Meyers Coupling, A. I. Meyers, E. D. Mihelich, J.

Am. Chem. Soc. 97 (1975) 7383; Suzuki Coupling, US-A-5130439 und DE-C-19607135 beschrieben.

Bevorzugt wird es für die Verseifung von Biphenylnitrilen der Formel (l) verwendet:

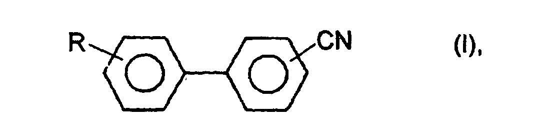

in der R ein Wasserstoffatom, einen Alkylrest, einen OAlkylrest, einen gegebenenfalls substituierten OArylrest, insbesondere eine substituierte oder unsubstituierte OPhenylgruppe, OH, F, Cl, Br oder 4-Phenylmethoxy bedeutet, wobei der Ausdruck "Alkyl" grundsätzlich jegliche Art von substituierten oder nicht substituierten Alkyl- oder Cycloalkylresten mit 1 bis 30 bzw. 3 bis 25 Kohlenstoffatomen umfaßt und das Biphenylgrundgerüst weiter substituiert sein kann.

Das in WO 97/30970 beschriebene Verfahren eignet sich beispielsweise für die Herstellung von unsymmetrisch substituierten Biphenylcarbonsäuren der Formel (l) mit beliebigem Substitutionsmuster bezüglich der Stellung der Substituenten (ortho, meta oder para), wobei das Biphenylgrundgerüst weiter substituiert sein kann.

Beispiele von Verbindungen, die mit dem erfindungsgemäßen Verfahren verseift werden können, sind 4'-Methoxybiphenyl-4-nitril, 4'-Methoxybiphenyl-4-nitril, 3'-Methoxybiphenyl-4-nitril, 4'-Hydroxybiphenyl-4-nitril, 4'-Oktyloxybiphenyl-4-nitril, 4'-Chlorbiphenyl-4-nitril, 4'-(Phenylmethoxy)biphenyl-4-nitril, 3,2',5'-Trimethybiphenyl-4-nitril und in 2', 3' bzw. 4'-Stellung mit einer Methylgruppe substituierte Biphenylnitrile, wie 2'-Methylbiphenyl-4-nitril, 4'-Methylbiphenyl-4-nitril, 4'-Methylbiphenyl-3-nitril und insbesondere 4'-Methylbiphenyl-2-nitril.

Als Verseifungsreagenzien können erfindungsgemäß Basen (basische Verseifung) oder Mineralsäuren (saure Verseifung) verwendet werden.

Als Basen kommen grundsätzlich alle Basen in Betracht, die üblicherweise bei der basischen Verseifung von Nitrilen verwendet werden (siehe beispielsweise Houben Weyl, Methoden der org. Chemie, Bd. E 5, S. 263 - 268; Bd. VIII, S. 426 - 433 und dort zitierte Literatur). Bevorzugt verwendet werden Basen der Formel M(OH)ₙ, in der M ein Alkali- und Erdalkalimetall ist und n 1 oder 2 ist. Beispiele für Alkalimetallbasen sind Lithiumhydroxid, Natriumhydroxid, Kaliumhydroxid, Rubidiumhydroxid und Cäsiumhydroxid. Beispiele für Erdalkalimetallbasen sind Berylliumhydroxid, Magnesiumhydroxid, Calciumhydroxid, Strontiumhydroxid und Bariumhydroxid. Aufgrund seiner leichten Verfügbarkeit und seines niedrigen Preises ist Natriumhydroxid die am stärksten bevorzugte Base.

Als Säuren kommen grundsätzlich ebenfalls alle Säuren in Betracht, die üblicherweise für die saure Verseifung von Nitrilen verwendet werden (siehe beispielsweise Houben Weyl, Methoden der org. Chemie, Bd. E 5, S. 263 - 268; Bd. VIII, S. 426 - 433 und dort zitierte Literatur). Bevorzugt verwendet werden (konzentrierte) Halogenwasserstoffsäuren, insbesondere Bromwasserstoffsäure und Chlorwasserstoffsäure, oder (hochprozentige) Schwefelsäure.

Diese Verseifungsreagenzien werden in üblichen Konzentrationen eingesetzt, wobei die optimale Konzentration von Verseifungsreagenz zu Biphenylnitril durch Reaktionskontrolle mittels HPLC bestimmt werden kann. Beispielsweise wird, wenn Natriumhydroxid als Verseifungsreagenz verwendet wird, die Konzentration derart gewählt, daß das jeweilige Carbonsäurenatriumsalz in der Hitze (etwa 90 °C) noch löslich ist. Für die alkalische Verseifung von in 2', 3' bzw. 4'-Stellung mit einer Methylgruppe substituierten Biphenylnitrilen, insbesondere 4'-Methylbiphenyl-2-nitril, mit Natriumhydroxid hat sich beispielsweise eine 50 %-ige Natriumhydroxidlösung als vorteilhaft erwiesen, wobei pro mol Biphenylnitril mehr als 1 mol, bevorzugt etwa 1,3 Mol Natriumhydroxid eingesetzt werden.

Augrund der Vielzahl von Reagenzien und Biphenylnitrilen, die eingesetzt werden können, kann die Temperatur ebenso wie die Konzentration der Reagenzien in einem weiten Bereich variieren.

Wird das Verfahren der vorliegenden Erfindung beispielsweise für die Verseifung von in 2', 3' bzw. 4'-Stellung mit einer Methylgruppe substituierten Biphenylnitrilen, insbesondere 4'-Methylbiphenyl-2-nitril, verwendet, wird es im allgemeinen bei einer Temperatur von 110 bis 190 °C, bevorzugt 130 bis 180 °C und stärker bevorzugt 150 bis 170 °C und bei einem Druck von 3 bis 20 bar, bevorzugt 3 bis 15 bar, stärker bevorzugt 5 bis 10 bar ausgeführt.

Als besonders vorteilhaft hat sich die alkalische Verseifung von 4'-Methylbiphenyl-2-nitril bei einer Temperatur von 150 - 155 °C und bei einem Druck von 3 bis 5 bar mit Natriumhydroxid erwiesen, die, nachdem 36 Stunden erhitzt worden ist, in nahezu quantitativer Ausbeute (98,7 %) zu einem Produkt mit hoher Reinheit (> 99,5 %) führt. Eine schnellere Umsetzung kann durch Steigern der Temperatur und/oder des Drucks erreicht werden, wobei Ausbeute, Selektivität und Reaktionsgeschwindigkeit durch Reaktionskontrolle mittels HPLC optimal aufeinander abgestimmt werden können.

Die Reaktion wird grundsätzlich in druckbeständigen Behältern ausgeführt, die säure- bzw. basenbeständig sind. Beispielsweise kann die Umsetzung in einem Bombenrohr oder in einem Autoklaven erfolgen, wobei bevorzugt ein Autoklav verwendet wird, der mit Mitteln zum Rühren, Mitteln zum Heizen, Mitteln zur Temperatur- und Druckkontrolle und einer Ablaßleitung ausgestattet ist (siehe beispielsweise Organikum, Johann Ambrosius Barth Verlag, Heidelberg - Leipzig, 1996, S. 17-19; Ullmanns Encyclopädie der technischen Chemie, 4. Auflage, Verlag Chemie Weinheim 1972, S. 14 ff.). Es ist beispielsweise im Fall der alkalischen Verseifung vorteilhaft, das entstehende Ammoniakgas kontinuierlich über einen Abgaskühler (z.B. Rückflußkühler, aufsteigendes gekühltes Rohr) mit Druckhalteventil abzulassen.

Die Reaktanden werden unter Schutzgas, beispielsweise Stickstoff, in dem druckbeständigen Behälter vorgelegt, der druckbeständige Behälter wird geschlossen und auf die gewünschte Temperatur erhitzt, wobei der Druck ansteigt. Wird ein druckbeständiger Behälter mit Mitteln zum Steuern des Drucks eingesetzt, kann der Druck durch Entspannen auf dem angestrebten Niveau gehalten werden.

Die Aufarbeitung erfolgt nach üblichen Verfahren. Beispielsweise kann im Fall der Umsetzung von 4'-Methylbiphenyl-2-nitril mit 50 %-igem Natriumhydroxid nach nahezu quantitativer Umsetzung des Nitrils zur Carbonsäure (HPLC-Kontrolle), die Reaktionslösung angesäuert werden (z.B. auf einen pH-Wert von 4), bei dem das Produkt, 4'-Methylbiphenyl-2-carbonsäure, ausfällt. Das Verfahren zeichnet sich in der Regel durch vollständige Umsetzung des Nitrils zur Carbonsäure aus. Reste von Carbonsäureamid, das bei der Verseifung von Nitril zur Carbonsäure als Zwischenstufe durchlaufen wird, können jedoch gegebenenfalls einfach durch Filtration und/oder Extraktion vor dem Ansäuern der Carbonsäure-Natriumsalzlösung abgetrennt werden. Nach Abtrennung des Niederschlags und dessen Trocknung erhält man dann das Produkt in 98,7 % Ausbeute und 99,5 % Reinheit. Ist ein Produkt höherer Reinheit erforderlich, kann das Produkt mittels üblicher Verfahren, wie Umkristallisation, Chromatographie etc., weiter gereinigt werden.

Biphenylcarbonsäuren, insbesondere 4'-Methylbiphenyl-2-carbonsäure, sind wichtige Zwischenprodukte bei der Herstellung von Flüssigkristallen und pharmazeutisch wirksamen Verbindungen, insbesondere Angiotensin-II-Inhibitoren (siehe beispielsweise CHIMICA OGGI/Chemistry Today, März/April 1998, S. 18 - 23 und dort zitierte Literaturstellen), die ein Biphenylgerüst enthalten.

Die Erfindung wird in dem folgenden Beispiel näher erläutert.

### Beispiel

### Herstellung von 4'-Methylbiphenyl-2-carbonsäure (MBC)

In einen 1 Liter Stahlautoklaven, der mit einem mechanischen Rührer, einem Heizmantel, einem Innenthermometer, einer Druckanzeige und einem Abgaskühler mit Druckhalteventil, das auf 5 bar eingestellt ist, ausgestattet ist, werden 193,2 g 4'-Methylbiphenyl-2-nitril (o-Toluylbenzonitril (OTBN), 1 mol), 345 g entsalztes Wasser und 104 g 50 %-iges Natriumhydroxid (1,3 mol) gegeben. Der Autoklav wird geschlossen und 5 mal mit Stickstoff gespült. Es wird ein Restdruck von 1 bar belassen und unter Rühren auf 150 bis 155 °C erwärmt. Dabei entsteht ein langsamer Druckanstieg. Bei 5 bar entweicht Ammoniakgas über das Druckhalteventil. Nach 36 Stunden wird eine Inprozeßkontrolle durchgeführt (HPLC). Wenn die Amidmenge kleiner als 0,1 % ist, wird der Ansatz aufgearbeitet.

Hierzu wird auf 60 °C abgekühlt, 5 g Aktivkohle (Carbopal SC 11) zugegeben, 1 Stunde gerührt und anschließend filtriert. Das Filtrat wird unter Rühren mit 471,8 g 10 %-iger Salzsäure (1,29 mol) versetzt. Ab einem pH-Wert von 6,7 entsteht eine weiße Suspension. Der End-pH-Wert beträgt 4. Die entstandene Suspension wird filtriert. Das weiße Feuchtprodukt wird gründlich mit Wasser gewaschen und 15 Stunden bei 50 °C im Vakuum getrocknet. Es werden 208,8 g (Ausbeute: 98,7 % der Theorie) weißes Produkt mit 99,5 % Reinheit erhalten.

## Patentansprüche

1. Verfahren zur alkalischen oder sauren Verseifung von Biphenylnitrilen, **dadurch gekennzeichnet, daß** die Verseifung in Wasser und unter einem Druck von 3 bis 20 bar ausgeführt wird.

2. Verfahren nach Anspruch 1, wobei die Biphenylnitrile solche der Formel (l) 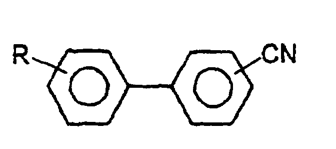 sind, in der R ein Wasserstoffatom, einen Alkylrest, einen OAlkylrest, einen gegebenenfalls substituierten OArylrest, OH, F, Cl, Br oder 4-Phenylmethoxy bedeutet.

3. Verfahren nach Anspruch 2, wobei die Verbindung der Formel (l) 4'-Methylbiphenyl-2-nitril ist.

4. Verfahren nach einem der Ansprüche 1 bis 3, wobei die Verseifung in Gegenwart einer Base der folgenden Formel M(OH)ₙ ausgeführt wird, wobei M ein Alkali- oder Erdalkalimetall ist und n 1 oder 2 ist.

5. Verfahren nach Anspruch 4, wobei die Base Natriumhydroxid ist.

6. Verfahren nach Anspruch 3 und 5, wobei die Verseifung bei einer Temperatur von 150 bis 155 °C und einem Druck von 3 bis 5 bar ausgeführt wird.

7. Verfahren nach einem der Ansprüche 1 bis 3, wobei die Verseifung in Gegenwart einer Säure ausgeführt wird, die aus Halogenwasserstoffsäuren, insbesondere Chlorwasserstoffsäure und Bromwasserstoffsäure, und Schwefelsäure ausgewählt ist.

## Claims

1. Method for the alkaline or acidic saponification (hydrolysis) of biphenylnitriles, **characterized in that** the saponification (hydrolysis) is carried out in water under pressure.

2. Method according claim 1, in the course of which the biphenylnitriles are such according the formula (I), 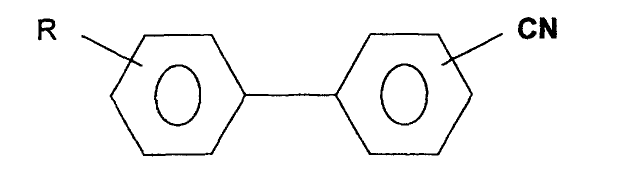 in which R is a hydrogenatom, an alkylgroup, an Oalkylgroup, a substituted or unsubstituted OArylgroup, OH, F, Cl, Br or 4-Phenylmethoxy.

3. Method according claim 2, in course of which the compound of formula (I) is 4'-methylbiphenyl-2-nitrile.

4. Method according one of the claims 1 to 3, in the course of which the saponification (hydrolysis) is carried out in presence of a base of formula M(OH)n, in which M is a alkali- or earthalkali-metal and n is 1 or 2.

5. Method according claim 4, wherein the base is sodium hydroxide.

6. Method according 3 and 5, wherein the saponification (hydrolysis) is carried out at a temperature of 150 to 155 °C under a pressure of 3 to 5 bar.

7. Method according one of the claims 1 to 3, in the course of which the saponification (hydrolysis) is carried out in presence of an acid, which is selected from hydrogenhalogenids, especially hydrochloric acid and hydrobromic acid, and sulfuric acid.

## Revendications

1. Méthode de saponification en milieu alcalin ou acide (hydrolyse) des biphénylnitriles, **caractérisée par le fait que** la saponification (hydrolyse) est réalisée en milieu aqueux sous pression.

2. Méthode de la revendication N°1, selon laquelle les biphenylnitriles utilisés ont la formule (I) développée suivante : 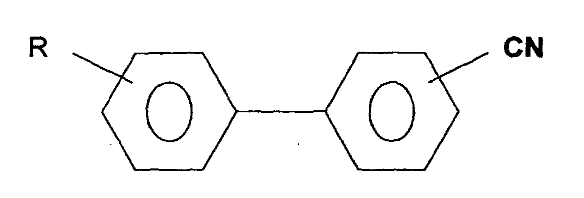 Dans laquelle R peut être un hydrogène (H), un groupement alkyl, un groupement alkyl oxygéné, un groupement alkyl oxygéné substitué ou non substitué, un hydroxyle (-OH), un halogène (F, Cl, Br) ou 4-méthoxyPhényl.

3. Méthode de la revendication N°2, au cours de laquelle le composé de formule (l) utilisé est le 4'-méthylbiphényl-2-nitrile.

4. Méthode selon les revendications 1 à 3, au cours de laquelle la saponification (hydrolyse) se déroule en présence d'une base dont la formule est M(OH)n, dans laquelle M est un métal alcalin ou alcalino-terreux et la valeur de n est 1 ou 2.

5. Méthode selon les revendications 4, dans laquelle la base est l'hydroxyde de sodium.

6. Méthode selon les revendications 3 à 5, dans laquelle la saponification (hydrolyse) est réalisée à une température de 150 à 155 °C sous une pression de 3 à 5 bar.

7. Méthode selon l'une des revendications 1 à 3, dans laquelle la saponification (hydrolyse) est réalisée en présence d'un acide qui peut être halogéné, et plus particulièrement les acides chlorhydrique et bromhydrique, et l'acide sulfurique.
